Europäisches Patentamt

European Patent Office

Office européen des brevets

(11) Veröffentlichungsnummer: **0 444 490 A2**

# EUROPÄISCHE PATENTANMELDUNG

(21) Anmeldenummer: **91102224.2**

(22) Anmeldetag: **16.02.91**

(51) Int. Cl.5: **A61M 1/00, A61M 5/00, A61B 5/14**

(30) Priorität: **24.02.90 DE 9002200 U**

(43) Veröffentlichungstag der Anmeldung:
**04.09.91 Patentblatt 91/36**

(84) Benannte Vertragsstaaten:
**AT CH DE ES FR GB GR IT LI NL**

(71) Anmelder: **Almo Erzeugnisse Erwin Busch GmbH**
**Grosse Allee 84**
**W-3548 Arolsen(DE)**

(72) Erfinder: **Ritter, Franz-Peter, Dipl.-Ing.**
**Schwabenstrasse 50-54,**
**W-8933 Untermeitingen(DE)**

(74) Vertreter: **Selting, Günther, Dipl.-Ing. et al**
**Patentanwälte von Kreisler, Selting, Werner**
**Deichmannhaus am Hauptbahnhof**
**W-5000 Köln 1(DE)**

(54) **Blutentnahmegerät.**

(57) Das Blutentnahmegerät besteht aus einem Blutentnahmeröhrchen (1) mit Schraubkappe (2), die einen Ansatz (3) zur Aufnahme eines Nadeladapters aufweist, einem im Röhrchen (1) geführten Kolben (4) und einer mit dem Kolben verbundenen Kolbenstange (5). An der Verbindungsstelle mit dem Kolben weist die Kolbenstange (5) eine Soll-Bruchstelle (5.1) auf. An dem Blutentnahmeröhrchen (1) sind zwischen vorderster und hinterster Kolbenstellung Markierungen (1a,1b) vorgesehen, die jeweils Teilvolumina des vollen Röhrchenvolumens angeben. Die Kolbenstange weist mindestens eine weitere Soll-Bruchstelle (5.2,5.3) auf, die jeweils einer Markierung (1a,1b) zugeordnet ist. Dadurch ist es möglich, auch Teilmengen von Blut aufzunehmen, die nicht dem vollen Röhrchenvolumen entsprechen.

Fig. 1

Fig. 2

Die Erfindung betrifft ein Blutentnahmegerät nach dem Oberbegriff des Anspruchs 1.

Derartige Blutentnahmegeräte sind allgemein bekannt und werden in verschiedenen Größen gefertigt, um unterschiedlich große Blutmengen vom Patienten entnehmen zu können, je nach Notwendigkeit für die betreffende Untersuchung.

Trotzdem findet im praktischen Betrieb die Verwendung unterschiedlicher Größen von Blutentnahmegeräten der gleichen Art bzw. Präparation vielfach nicht statt. Einer der dafür maßgeblichen Gründe liegt darin, daß man angesichts der Vielzahl von mit unterschiedlichen Präparationen versehenen Arten von Blutentnahmegeräten und in dem Bestreben nach Überschaubarkeit der verfügbar gehaltenen Geräte die Anzahl der bevorrateten und zum Gebrauch bereitgehaltenen Geräte nicht noch dadurch vergrößern will, daß von den verschiedenen Blutentnahmegerät-Arten jeweils auch noch mehrere verschiedene Größen verwendet werden. Ein anderer der maßgeblichen Gründe kann darin liegen, daß, namentlich bei Auswertung der Untersuchungen im hauseigenen Labor, man möglichst mit einer Röhrchengröße der Blutentnahmegeräte auskommen will, um nicht ständig wechselnde Zentrifugeneinsätze usw. verwenden zu müssen. Arbeitet man aber nur mit einer Röhrchengröße der Blutentnahmegeräte, entscheidet man sich natürlich für die größte Röhrchengröße, damit auch die Untersuchungen durchgeführt werden können, für die ein größeres Blutvolumen notwendig ist.

Andererseits zeigt aber die Erfahrung, daß die Blutabnahme, namentlich in Krankenhäusern und bei Krankheitsverläufen, bei denen eine mehrfache Blutabnahme erforderlich ist, eine erhebliche Belastung für den ohnehin geschwächten Organismus des Patienten darstellt. Deshalb ist es vom medizinischen Standpunkt unzweckmäßig, dem Patienten zur Untersuchung mehr Blut abzunehmen, als tatsächlich unbedingt erforderlich ist.

Da die Blutentnahmegeräte üblicherweise mit einer Skalierung versehen sind, ist es zwar grundsätzlich möglich, auch mit größeren Blutentnahmegeräten jeweils nur eine Teilmenge Blut abzunehmen, beispielsweise bei einem 10-ml-Blutentnahmeröhrchen nur eine Teilmenge von 3 ml oder 6 ml. Allerdings stellt sich bei einer solchen Teilmengenentnahme das Problem, daß die Kolbenstange vom Kolben getrennt werden muß, denn das Entnahmeröhrchen dient ja gleichzeit als Analyseröhrchen und muß in ein Analysegerät oder eine Zentrifuge eingesetzt werden, wo die unten über das Röhrchenende überstehende Kolbenstange hinderlich wäre. Eine solche Trennung der Kolbenstange vom Kolben ist nur dann möglich, wenn eine Schraubverbindung zwischen Kolbenstange und Kolben vorgesehen ist. Meist ist aber eine Sollbruchstelle an der Verbindung der Kolbenstange mit dem Kolben vorhanden, und dann läßt sich die Kolbenstange aber nur dann vom Kolben trennen, wenn sich der Kolben in hinterster Stellung befindet. In diesem Fall sind also Teilmengenentnahmen praktisch gar nicht möglich, es sei denn, daß nach der eigentlichen Blutentnahme noch weiter aufgezogen wird und so das Restvolumen des Röhrchens mit Luft gefüllt wird, was aber auch nicht wünschenswert ist, denn dann wäre der Flüssigkeitsspiegel im Röhrchen zu tief, um mit üblichen Analysegeräten Proben aus den Röhrchen zu entnehmen. Außerdem ist bei Blutentnahmegeräten, die eine beim Trennen von der Nadel selbstschließende Kappe aufweisen, ein weiteres Aufziehen des Kolbens gar nicht möglich. Nach der Blutentnahme wird das Röhrchen außerdem üblicherweise mit einem die notwendigen Patientendaten enthaltenden Etikett überklebt, das den gesamten Röhrchenumfang in Anspruch nimmt, mit der Folge des weiteren Problems, daß nachher nicht mehr erkennbar ist, wieviel Blut sich tatsächlich im Röhrchen befindet.

Der Erfindung liegt daher die Aufgabe zugrunde, ein Blutentnahmegerät der eingangs genannten Gattung so auszubilden, daß auch Teilmengen des Röhrchenvolumens entnommen werden können. Eine darauf aufbauende weitere Aufgabe besteht darin, auch bei durch Etikettierung zugeklebtem Entnahmeröhrchen bei der späteren labormäßigen Handhabung leicht feststellen zu können, welche Blutmenge sich tatsächlich in dem Röhrchen befindet.

Diese Aufgabe wird gemäß der Erfindung durch das im Anspruch 1 gekennzeichnete Blutentnahmegerät gelöst.

Das erfindungsgemäße Blutentnahmegerät nach Anspruch 2 löst zusätzlich auch noch die genannte weitere Aufgabe.

Weitere bevorzugte Ausgestaltungen der Erfindung sind Gegenstand der Ansprüche 3 und 4.

Das erfindungsgemäße Blutentnahmeröhrchen ermöglicht das Abbrechen der Kolbenstange auch in Zwischenpositionen entsprechend den vorgesehenen Teilmengenentnahmen und läßt gleichzeitig die jeweils entnommene Blutmenge anhand des Bruchbildes der abgebrochenen Kolbenstange ohne weiters erkennen, selbst wenn das Entnahmeröhrchen mit einem Etikett so zugeklebt ist, daß sein Inhalt nicht mehr sichtbar ist. Beim Abbrechen der Kolbenstange in einer Zwischenstellung stützt der verbleibende Rest der Kolbenstange den Kolben im Röhrchen so ab, daß er sich auch beim Zentrifugieren nicht unter der Fliehkrafteinwirkung verschieben kann. Eine solche unerwünschte Kolbenverschiebung kann nämlich auftreten, wenn eine Kolbenstange bei einem herkömmlichen Gerät mit Schraubverbindung zwischen Kolbenstange und Kolben in einer Zwischenstellung des Kolbens einfach herausgeschraubt würde.

Die Erfindung wird nachstehend unter Bezugnahme auf die anliegenden Zeichnungen mehr im einzelnen beschrieben, in denen zeigen:

Fig. 1

das Blutentnahmegerät in schematisierter Darstellung,

Fig. 2

den Kolben mit der Kolbenstange des Blutentnahmegeräte und

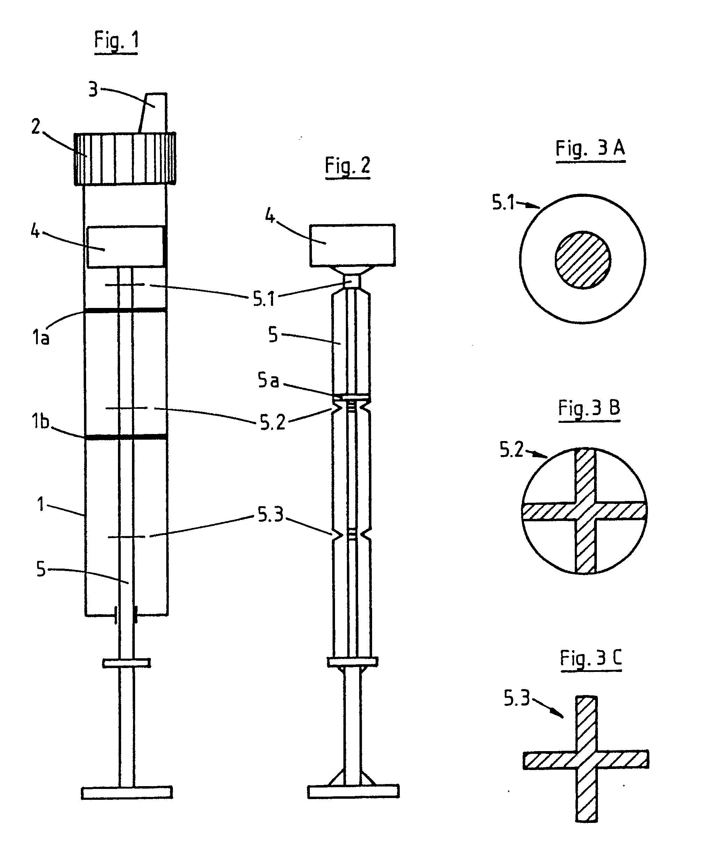

die Fig. 3A bis 3C

die Bruchstellenquerschnitte der Kolbenstange entsprechend der jeweils aufgezogenen Blutmenge.

Fig. 1 zeigt in schematisierter Seitenansicht das Blutentnahmegerät mit einem 10 ml-Röhrchen. Das Gerät besteht aus dem Blutentnahmeröhrchen 1 mit aufgeschraubter Schraubkappe 2, die einen Ansatz 3, beispielsweise Luer-Konus, zum Aufsetzen eines Nadeladapters aufweist, einem im Röhrchen geführten Kolben 4 und der damit verbundenen, nach hinten aus dem Röhrchen 1 herausragenden Kolbenstange 5.

Das Röhrchen 1 trägt eine aufgedruckte Skalierung, die hier nicht im einzelnen dargestellt ist. Lediglich zwei besondere Marken 1a und 1b für aufgezogenen Blutmengen von 3 ml bzw. 6 ml der Skalierung sind dargestellt. Mit dem Gerät können also wahlweise durch Aufziehen des Kolbens bis zu einer der beiden Markierungen 1a und 1b bzw. zum hinteren Anschlag drei verschiedene Blutmengen von 3 ml, 6 ml und 10 ml aufgezogen werden.

Da das Blutentnahmeröhrchen gleichzeitig als Analyseröhrchen dient, muß nach dem Aufziehen des Blutes die Kolbenstange, so weit sie aus dem hinteren Röhrchenende übersteht, entfernt werden, damit das Röhrchen in Ständer, Zentrifugeneinsätze etc. eingesetzt werden kann. Dazu hat die Kolbenstange 5 an ihrer Verbindung mit dem Kolben 4, wie allgemein üblich, eine Sollbruchstelle 5.1. Zusätzlich hat die Kolbenstange 5 aber in ihrem Verlauf noch zwei weitere Sollbruchstellen 5.2 und 5.3, die jeweils an denjenigen Positionen entlang der Länge der Kolbenstange 5 gelegen sind, die beim Aufziehen des Kolbens 4 bis zur Markierung 1b bzw. 1a, also zum Aufziehen von Teilmengen von Blut, jeweils am hinteren Röhrchenende liegen. Die Kolbenstange kann also nach dem Aufziehen von nur 3 ml oder 6 ml Blut jeweils an der betreffenden Sollbruchstelle 5.3 bzw. 5.2 teilweise abgebrochen werden.

Wie Fig. 2 zeigt, hat die Kolbenstange an der Sollbruchstelle 5.1, also an ihrer Verbindung mit dem Kolben 4, einen verhältnismäßig dünnen zylindrischen Querschnitt; über den größten Teil ihrer verbleibenden Länge hat sie ein kreuzförmiges Querschnittsprofil. An der hintersten Sollbruchstelle 5.3 ist das Kreuzprofil zur Bildung der Sollbruchstelle entsprechend geschwächt, indem die vier Kreuzarme eingekerbt sind. Gleiches gilt für die mittlere Sollbruchstelle 5.2, wobei allerdings, vom Kolben aus gesehen, unmittelbar vor dieser Sollbruchstelle ein Ringbund 5a gebildet ist.

Demgemäß ergeben sich beim Abbrechen der Kolbenstange an den drei verschiedenen Sollbruchstellen 5.1, 5.2 und 5.3 die in den Fig. 3A bis 3C gezeigten Bruchbilder:

a) An der Sollbruchstelle 5.1 hat der Bruchquerschnitt die Form eines runden Punktes, da dort die Kolbenstange einen dünnen zylindrischen Querschnitt hat.

b) An der zweiten Sollbruchstelle hat der Bruchquerschnitt, von hinten gesehen, die Form eines Kreuzes (wegen des kreuzförmigen Querschnittsprofils der Kolbenstange) auf einer Kreisfläche, nämlich der Stirnfläche des Ringbundes 5a unmittelbar am Bruchquerschnitt jener Sollbruchstelle.

c) An der hintersten Sollbruchstelle hat der Bruchquerschnitt nur die Form eines einfachen Kreuzes entsprechend dem Kreuzprofil der Kolbenstange.

Ist nun das ganz oder teilweise mit Blut gefüllte Röhrchen 1 durch Etikettierung so zugeklebt, daß man seinen Inhalt nicht mehr erkennen kann, ist anhand des Bruchbildes der abgebrochenen Kolbenstange, das für jede der drei Sollbruchstellen, also entsprechend für jede der drei verschiedenen Füllmengen des Röhrchens, unterschiedlich ist, leicht erkennbar, wieviel Blut sich im Röhrchen befindet. Der Bruchquerschnitt selbst hebt sich durch seine Farbe, die intensiver als die der sonst glatten Kunststoffoberflächen der Kolbenstange ist, deutlich ab und ist deshalb leicht identifizierbar.

**Patentansprüche**

1. Blutentnahmegerät, bestehend aus einem Blutentnahmeröhrchen (1) mit Schraubkappe (2), die einen Ansatz (3) zur Aufnahme eines Nadeladapters aufweist, einem im Röhrchen (1) geführten Kolben (4) und einer mit dem Kolben verbundenen und aus dem hinteren Röhrchenende herausragenden Kolbenstange (5), wobei die Kolbenstange an der Verbindungsstelle mit dem Kolben eine Sollbruchstelle (5.1) aufweist, dadurch gekennzeichnet, daß das Blutentnahmeröhrchen (1) zwischen vorderster und hinterster Kolbenstellung mindestens eine einem Teilvolumen des verfügbaren Röhrchenvolumens bei vollständig zurückgezogenem Kolben entsprechende Markierung (1a, 1b) trägt und die Kolbenstange mindestens eine weitere Sollbruchstelle (5.2, 5.3) aufweist, die bei bis zu der Markierung (1a, 1b) zurückgezogenem Kolben (4) am hinteren Röhrchenende liegt.

2. Blutentnahmegerät nach Anspruch 1, dadurch gekennzeichnet, daß jede Sollbruchstelle (5.1, 5.2, 5.3) durch unterschiedliche konstruktive Gestaltung ein von jeder anderen Sollbruchstelle verschiedenes Bruchquerschnittsbild (Fig. 3A bis 3C) erzeugt.

3. Blutentnahmegerät nach Anspruch 1 oder 2, dadurch gekennzeichnet, daß das Röhrchen (1) zwei Teilvolumen-Markierungen (1a, 1b) trägt und die Kolbenstange zwei weitere Sollbruchstellen (5.2, 5.3) aufweist.

4. Blutentnahmegerät nach den Ansprüchen 2 und 3, dadurch gekennzeichnet, daß die Kolbenstange an ihrer Verbindung (5.1) mit dem Kolben (4) einen dünnen zylindrischen Querschnitt, an der zweiten Sollbruchstelle (5.2) einen kreuzförmigen Querschnitt mit einem an der kolbenseitigen Seite der Sollbruchstelle unmittelbar angrenzenden Ringbund, und an der dritten Sollbruchstelle (5.3) einen nur kreuzförmigen Querschnitt aufweist.

Fig. 1

Fig. 2

Fig. 3 A

Fig. 3 B

Fig. 3 C